# EUROPEAN PATENT APPLICATION

(11) **EP 3 933 391 A1**
(43) Date of publication of application: **05.01.2022**
(21) Application number: 21769622.8
(22) Date of filing: 06.05.2021
(51) Int. Cl.: G01N 23/223

(54) **X-RAY FLUORESCENCE ANALYZER**

(30) Priority: 08.05.2020 KR 20200054843
(71) Applicant: ISP Co., Ltd., Jeonju-si Jeollabuk-do 54853 (KR)
(72) Inventor: PARK, Jeong Goun, Jeonju-si, Jeollabuk-do 54853 (KR)
(74) Representative: Cabinet Chaillot
(86) International application number: PCT/KR2021/005671
(87) International publication number: WO 2021/225381

(57) **Abstract**

The present disclosure relates to an X-ray fluorescence analyzer, which includes a sample box configured to accommodate a liquid sample; an X-ray generation unit configured to irradiate an X-ray to one side surface of the inside of the sample box; and a detector disposed along one side surface of the sample box at which a distance of a fluorescent X-ray emitted from the inside of the sample box to the outside of the sample box is shortest in order to minimize absorption of the fluorescent X-ray emitted out of the sample box in the air, when the X-ray irradiated by the X-ray generation unit reacts with the liquid sample inside the sample box to emit the fluorescent X-ray out of the sample box, the detector being configured to detect the fluorescent X-ray.

According to the present disclosure, a target element may be measured more precisely in an atmospheric environment without vacuum exhaust by irradiating an X-ray in a state as close to an inner wall of a sample box as possible to increase an emitted fluorescent X-ray while minimizing absorption by a liquid.

In addition, by using a structure in which a secondary target is disposed and characteristic X-ray generated from the target is irradiated to the sample again, the radiation that is inevitably discarded may be returned back to the inside of the sample box, thereby increasing the efficiency.

## Description

### [Technical Field]

This disclosure relates to an X-ray fluorescence analyzer for measuring elements contained in a liquid sample, and more specifically, to an X-ray fluorescence analyzer that may measure a target element in an atmospheric environment without vacuum exhaust by irradiating an X-ray in a state as close to an inner wall of a sample box as possible to increase an emitted fluorescent X-ray while minimizing absorption by a liquid.

### [Background Art]

Recently, in accordance with the global demand for environmentally friendly products and the increase in the resource circulation-type structure of consumption, measurement devices capable of qualitatively/quantitatively analyzing materials in all production processes are being developed.

Among them, an X-ray fluorescence analysis device using an X-ray may analyze a material to be measured in a non-destructive way to minimize the pretreatment process of the material, and acquire information about the material to be measured in a short time, so it is a technology widely used in product production and import inspection by irradiating an X-ray to a sample and then detecting a fluorescent X-ray generated from the sample.

Meanwhile, the upper limit is determined with respect to the sulfur concentration in fuel oils, such as gasoline, diesel and kerosene, and sulfur concentration in a fuel oil is measured, managed and controlled during or after refinement of crude oil. In recent years, as regulation has been strengthened even in Europe, America and Japan, the upper limit of the standard is being lowered.

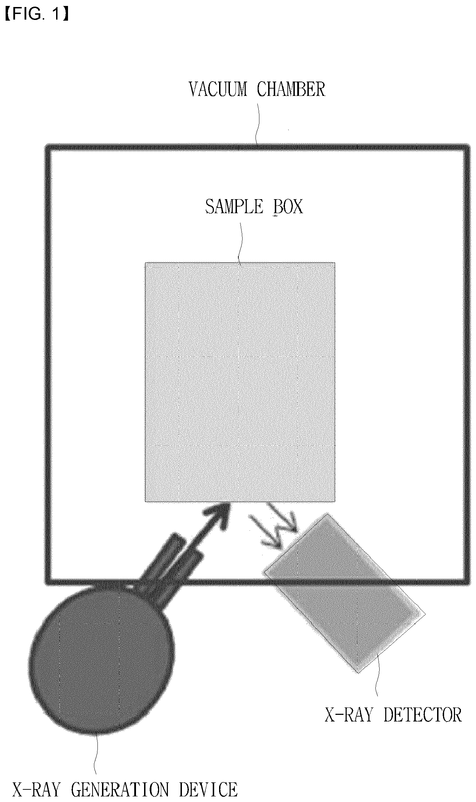

If a light element such as sulfur is analyzed using a common X-ray for the sulfur concentration, a vacuum chamber must be prepared to make the measurement environment vacuous and a sample must be placed inside the vacuum chamber as shown in FIG. 1 in order to minimize absorption by oxygen and nitrogen in the air. Here, it takes a long time to form a vacuum in the vacuum chamber.

In addition, in the process of irradiating an X-ray, since the fluorescent X-ray expressed inside the sample box is disposed far away from the outer side of the sample box, if the expressed fluorescent X-ray does not have sufficient energy, the generated fluorescent X-ray does not penetrate to the outside, which may make it difficult to accurately analyze the elements.

### [Disclosure]

### [Technical Problem]

This disclosure is directed to providing an X-ray fluorescence analyzer, which may measure a target element in an atmospheric environment without vacuum exhaust by irradiating an X-ray in a state as close to an inner wall of a sample box as possible to increase an emitted fluorescent X-ray while minimizing absorption by a liquid.

The object of the present disclosure is not limited to the above, and other objects not mentioned herein will be clearly understood by those skilled in the art from the following disclosure.

### [Technical Solution]

In one general aspect, there is provided an X-ray fluorescence analyzer, comprising: a sample box configured to accommodate a liquid sample; an X-ray generation unit configured to irradiate an X-ray to one side surface of the inside of the sample box; and a detector disposed along one side surface of the sample box at which a distance of a fluorescent X-ray emitted from the inside of the sample box to the outside of the sample box is shortest in order to minimize absorption of the fluorescent X-ray emitted out of the sample box in the air, when the X-ray irradiated by the X-ray generation unit reacts with the liquid sample inside the sample box to emit the fluorescent X-ray out of the sample box, the detector being configured to detect the fluorescent X-ray.

Here, the X-ray generation unit may be disposed at one side surface of the sample box to irradiate the X-ray in parallel to an inner wall of the sample box at which a distance of the fluorescent X-ray emitted toward the detector is shortest, among peripheral surfaces of the sample box.

Here, the X-ray generation unit may be disposed at one side surface of the sample box to irradiate the X-ray to be inclined at a predetermined angle toward an outer surface of the sample box at which a distance of the fluorescent X-ray emitted toward the detector is shortest, among peripheral surfaces of the sample box.

Here, the X-ray fluorescence analyzer may further comprise a secondary target disposed at the other side surface of the sample box where the X-ray generation unit is disposed to reflect the X-ray irradiated by the X-ray generation unit so that the reflected X-ray reacts with the liquid sample inside the sample box again to increase fluorescence expression of the X-ray.

Here, the secondary target may be made of titanium.

Here, the sample box may be coated with a resin film serving as an inner film.

### [Advantageous Effects]

The X-ray fluorescence analyzer according to the present disclosure may measure a target element in an atmospheric environment more precisely without vacuum exhaust by irradiating an X-ray in a state as close to an inner wall of a sample box as possible to increase an emitted fluorescent X-ray while minimizing absorption by a liquid.

In addition, by using a structure in which a secondary target is disposed and characteristic X-ray generated from the target is irradiated to the sample again, the radiation that is inevitably discarded may be returned back to the inside of the sample box, thereby increasing the efficiency.

The effect of the present disclosure is not limited to the above, and other effects not mentioned herein will be clearly understood by those skilled in the art from the following disclosure.

### [Description of Drawings]

FIG. 1 is a diagram showing a conventional light element measurement system.
FIG. 2 is a diagram showing an X-ray fluorescence analyzer for measuring elements included in a liquid sample according to an embodiment of the present disclosure.
FIG. 3 is a diagram showing an X-ray fluorescence analyzer for measuring elements included in a liquid sample according to another embodiment of the present disclosure.

### [Best Mode]

Hereinafter, a preferred embodiment of the present disclosure will be described in detail with reference to the accompanying drawings.

At this time, it should be noted that in the accompanying drawings, the same components are denoted by the same reference numerals as much as possible.

A detailed description of a known function or configuration that may obscure the gist of the present disclosure will be omitted.

For the same reason, some components are exaggerated, omitted or schematically illustrated in the accompanying drawings.

Throughout the specification, when any part "includes" a certain element, it means that another element may be further included therein, rather than excluding other elements, unless otherwise stated.

In addition, throughout the specification, when any part is disposed "on" a certain part, it means that the part is located above or below the target part, and does not necessarily mean that the part is located at an upper side based on the direction of gravity.

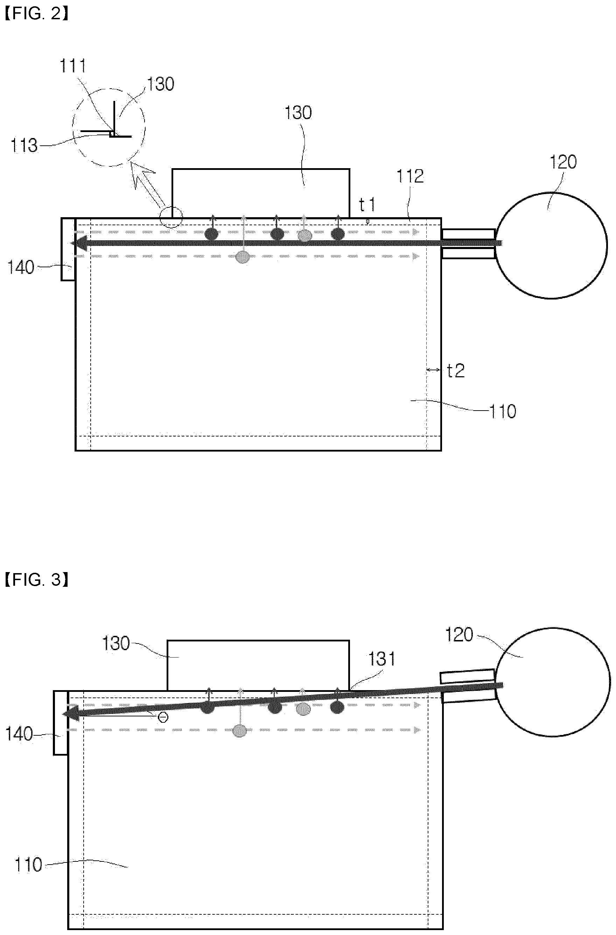

FIG. 2 is a diagram showing an X-ray fluorescence analyzer for measuring elements included in a liquid sample according to an embodiment of the present disclosure, and FIG. 3 is a diagram showing an X-ray fluorescence analyzer for measuring elements included in a liquid sample according to another embodiment of the present disclosure.

Referring to FIGS. 2 to 3, an X-ray fluorescence analyzer 10 for measuring a light element according to an embodiment of the present disclosure includes a sample box 110, an X-ray generation unit 120, a detector 130 and a secondary target 140.

The sample box 110 may be formed in an approximately rectangular parallelepiped shape and accommodate a liquid sample.

The liquid sample may be a variety of samples such as low sulfur gas oil, diesel and bunker C oil, but the sample is not limited thereto.

The inner peripheral surface of the sample box 110 may be airtight by being coated with a resin film.

The sample box 110 may prevent the sample box 110 from being damaged by an acid or alkali component that may be included in the liquid sample. The resin film may be made of a PE (polyethylene)-based resin, but the resin film is not limited thereto.

In addition, the resin film is preferably formed as a thin film so as not to prevent a fluorescent X-ray from being emitted out of the sample box 110.

At this time, a groove 111 may be formed at an outer peripheral surface of the sample box 110 in an adhesion region in order to increase the adhesion to the detector 130. In addition, a sealing material 113 may be provided along the circumference of the groove 111. Since the adhesion between the sample box 110 and the detector 130 is increased by means of the groove 111 and the sealing material 113 as above, it is possible to minimize the amount of radiation absorbed by the gas contained in the air, such as oxygen or nitrogen.

Therefore, it is possible to accurately measure elements in a liquid sample through a simpler system effectively without implementing a vacuum environment.

In addition, among the inner peripheral surfaces of the sample box 110, the thickness t1 of one side surface wall 112 of the sample box 110, at which the distance of the fluorescent X-ray emitted out of the sample box 110 is shortest, may be relatively smaller than the thickness t2 of the other walls.

Accordingly, the emission effect of the fluorescent X-ray to the outside of the sample box 110 may be further increased.

Although not shown in the drawings, an in-line may be implemented in the sample box 110 so that the liquid sample may be circulated periodically or aperiodically.

The in-line may increase the reliability of the measurement result by circulating the measurement sample as needed, and allow the liquid sample contained in the sample box 110 to be exchanged more easily.

This feature cannot be applied in an environment where vacuum exhaust is necessarily required.

The X-ray generation unit 120 irradiates an X-ray to one side surface of the inside the sample box 110.

The X-ray generation unit 120 may be disposed at one side surface of the sample box 110 so that the X-ray is irradiated in parallel to the inner wall 112 of the sample box 110 at which the distance of the fluorescent X-ray emitted toward the detector 130 is shortest among the peripheral surfaces of the sample box 110.

If the X-ray generation unit 120 is disposed at such a position to emit an X-ray, more fluorescent X-rays of the element to be measured are emitted through the inner wall 112 of the sample box 110 located closest to the outside, so it is possible to prevent the problem that the emitted X-ray is absorbed again by the liquid sample accommodated therein and cannot be emitted.

Conventionally, since light elements such as sulfur (S), phosphorus (P) and aluminum (Al) with low atomic numbers are well absorbed in the air, they are absorbed in the air before reaching the detector, and the fluorescence expressed from the inside is not released to the outside, preventing accurate measurement.

However, in the present disclosure, as described above, since more fluorescent X-rays may be emitted through the inner wall 112 of the sample box 110, it is possible to more accurately measure the measurement element without implementing a vacuum environment.

At this time, the X-ray generation unit 120 may be disposed so that an X-ray is vertically irradiated with respect to the outer wall of the sample box 110, as shown in FIG. 2.

Such an arrangement method is preferable for measuring a heavy metal having a relatively high atomic number relative to a light element, without being limited thereto.

In addition, as shown in FIG. 3, the X-ray generation unit 120 may be disposed at one side surface of the sample box so that an X-ray is irradiated to be inclined at a predetermined angle toward the outer wall of the sample box 110 at which the distance of the fluorescent X-ray emitted toward the detector 130 is shortest among the peripheral surfaces of the sample box 110.

At this time, the inclination angle of the X-ray generation unit 120 may not be inclined at the same angle as 45 degrees as shown in FIG. 1, but may be inclined at a relatively small angle compared to the angle.

Here, the predetermined angle θ means an angle at which the X-ray irradiated from the X-ray generation unit 120 is located below a bottom region 131 in contact with the detector 130 located adjacent to the X-ray generation unit 120 and is irradiated to be inclined to an upper side of a bottom region of the secondary target 140 disposed at the other side of the sample box 110 opposite to the X-ray generation unit 120.

If the irradiation angle of the X-ray generation unit 120 is arranged to be inclined as above, the X-ray irradiated into the sample box 110 may react with the sample located at the wall of the sample box 110 and then be emitted directly to the detector 130 disposed adjacent thereto, thereby making is possible to increase the elemental acquisition rate of the detector.

At this time, the wall of the sample box 110 to which the secondary target 140 is attached to correspond to the X-ray irradiated to be inclined by the predetermined angle θ may be formed to be inclined at a predetermined angle, thereby increasing the reflection efficiency of the secondary target 140.

If the X-ray generation unit 120 is arranged to be inclined with respect to the above-mentioned criteria, it is possible to more accurately measure light elements such as sulfur (S), phosphorus (P) and aluminum (Al) having low atomic numbers.

Meanwhile, although not shown in the drawings, a jig coupled with the X-ray generation unit 120 may be further included.

The jig may adjust the angle of the X-ray generation unit 120 so that the angle of the irradiated X-ray is variably adjusted according to the irradiation conditions of the X-ray generation unit 120.

Therefore, as described above, the irradiation position of the X-ray generation unit 120 may be optimized in consideration of the type of sample, the size and arrangement conditions of the sample box 110 and the detector 130, and the like.

The detector 130 may be disposed along one side surface of the sample box 110 at which the distance of the fluorescent X-ray emitted from the inside of the sample box 110 to the outside of the sample box 110 is shortest in order to minimize the absorption of the fluorescent X-ray emitted out of the sample box 110 in the air, when the X-ray irradiated by the X-ray generation unit 120 reacts with the liquid sample inside the sample box 110 to emit the fluorescent X-ray out of the sample box 110.

The detector 130 preferably has a light receiving surface formed on the same horizontal plane as one side surface of the sample box 110 at which the fluorescent X-ray has a shortest distance, and the light receiving surface is configured to be completely in close contact with one side surface of the sample box 110.

For example, sulfur (S) contained in diesel may be detected through the present disclosure.

When the X-ray generation unit 120 is irradiated to the inside of the sample box 110, the excited sulfur (S) may be obtained through one side surface wall 112 of the sample box at which the fluorescent X-ray of the sample box 110 has a shortest distance.

Meanwhile, if an X-ray is emitted at a large angle such as 45 degrees as in FIG. 1 of the prior art, there may be a problem that the amount of X-rays absorbed inside increases and the amount of measured fluorescent elements emitted outside decreases.

The secondary target 140 is disposed at the other side of the sample box 110 where the X-ray generation unit 120 is disposed.

The secondary target 140 may increase the expression of X-ray fluorescence by reflecting the X-ray irradiated from the X-ray generation unit 120 to react with the liquid sample inside the sample box again.

For example, if a target element to be detected is a light element such as sulfur (S), sulfur (S) requires energy of 231 keV.

At this time, the secondary target 140 may be made of titanium that is about twice of sulfur so as to emit a specific radiation to ideally excite sulfur.

Titanium emits a specific radiation (Ti = 451 keV) about twice compared with sulfur.

Therefore, the secondary target 140 may increase its efficiency by returning the radiation, which that has to be discarded, back to the inside of the sample box 110 again.

The secondary target 140 may be variously applied depending on the measurement element to be detected, and is not limited to titanium presented as an example above.

The X-ray fluorescence analyzer according to the present disclosure may measure a target element in an atmospheric environment more precisely without vacuum exhaust by irradiating an X-ray in a state as close to an inner wall of a sample box as possible to increase an emitted fluorescent X-ray while minimizing absorption by a liquid.

In addition, by using a structure in which a secondary target is disposed and characteristic X-ray generated from the target is irradiated to the sample again, the radiation that is inevitably discarded may be returned back to the inside of the sample box, thereby increasing the efficiency.

Meanwhile, the embodiments of the present disclosure illustrated in the specification and drawings are merely presented as specific examples to easily explain the technical content and help the understanding of the present disclosure, and are not intended to limit the scope of the present disclosure.

In addition to the embodiments disclosed herein, it is apparent to those skilled in the art that other modifications can be implemented based on the technical idea of the present disclosure.

## Claims

1. An X-ray fluorescence analyzer, comprising:
a sample box configured to accommodate a liquid sample;
an X-ray generation unit configured to irradiate an X-ray to one side surface of the inside of the sample box; and
a detector disposed along one side surface of the sample box at which a distance of a fluorescent X-ray emitted from the inside of the sample box to the outside of the sample box is shortest in order to minimize absorption of the fluorescent X-ray emitted out of the sample box in the air, when the X-ray irradiated by the X-ray generation unit reacts with the liquid sample inside the sample box to emit the fluorescent X-ray out of the sample box, the detector being configured to detect the fluorescent X-ray.

2. The X-ray fluorescence analyzer according to claim 1,
wherein the X-ray generation unit is disposed at one side surface of the sample box to irradiate the X-ray in parallel to an inner wall of the sample box at which a distance of the fluorescent X-ray emitted toward the detector is shortest, among peripheral surfaces of the sample box.

3. The X-ray fluorescence analyzer according to claim 1,
wherein the X-ray generation unit is disposed at one side surface of the sample box to irradiate the X-ray to be inclined at a predetermined angle toward an outer surface of the sample box at which a distance of the fluorescent X-ray emitted toward the detector is shortest, among peripheral surfaces of the sample box.

4. The X-ray fluorescence analyzer according to claim 1, further comprising:
a secondary target disposed at the other side surface of the sample box where the X-ray generation unit is disposed to reflect the X-ray irradiated by the X-ray generation unit so that the reflected X-ray reacts with the liquid sample inside the sample box again to increase fluorescence expression of the X-ray.

5. The X-ray fluorescence analyzer according to claim 4,
wherein the secondary target is made of titanium.

6. The X-ray fluorescence analyzer according to claim 1,
wherein the sample box is coated with a resin film serving as an inner film.
